# EUROPÄISCHE PATENTSCHRIFT

(11) **EP 1 146 335 B1**
(45) Veröffentlichungstag und Bekanntmachung des Hinweises auf die Patenterteilung: **02.06.2004**
(21) Anmeldenummer: 01108716.0
(22) Anmeldetag: 06.04.2001
(51) Int. Cl.: G01N 31/22, G01N 33/00, G01N 33/18

(54) **Verfahren für die Analyse von gasförmigen Inhaltsstoffen sowie Testkit insbesondere zur Durchführung dieses Verfahrens**
Method for analysing gaseous contents and test kit for carrying out the method
Méthode d'analyse du contenu en gaz et trousse d'essai pour la mise en oeuvre de cette méthode

(30) Priorität: 15.04.2000 DE 10018784
(43) Veröffentlichungstag der Anmeldung: 17.10.2001
(73) Patentinhaber: Macherey, Nagel GmbH & Co. Handelsgesellschaft, 52355 Düren (DE)
(72) Erfinder: Radmacher, Edmund Dr., 52349 Düren (DE); Möller, Klaus Dr., 52249 Eschweiler (DE); Niendieck, Fritz, 52078 Aachen (DE)
(74) Vertreter: Paul, Dieter-Alfred, Dipl.-Ing.

(56) Entgegenhaltungen:
- EP-A- 0 663 239
- DE-A- 2 712 158
- US-A- 4 315 890
- US-A- 5 320 807
- US-A- 5 979 219

## Beschreibung

Die Erfindung betrifft ein Verfahren für die Analyse eines gasförmigen oder in Gasform überführbaren Inhaltsstoffes einer Probe, bei dem die Probe über eine Behälteröffnung in einen Probeaufnahmebehälter eingefüllt und bei dem ein zuvor mit einem Indikatorreagenz versehener Analysenbehälter mit seiner Behälteröffnung über einen Adapter mit der Behälteröffnung des Probeaufnahmebehälters verbunden wird, wonach der Inhaltsstoff aus der Probe in den Analysenbehälter getrieben wird. Die Erfindung bezieht sich des weiteren auf einen Testkit insbesondere für die Durchführung des vorbeschriebenen Verfahrens mit einem Probeaufnahmebehälter für die Aufnahme der Probe über eine Behälteröffnung und mit einem Analysenbehälter für die Aufnahme des zu analysierenden Inhaltsstoffes über eine Behälteröffnung, wobei der Analysenbehälter ein Indikatorreagenz enthält oder mit einem Indikatorreagenz versehbar und als Meßvorlage in einem optischen Meßgerät verwendbar ist, sowie mit einem Adapter, über den die Behälteröffnungen miteinander verbindbar sind.

In der wasseranalytik ist es bekannt, aus einer Wasserprobe bestimmte Inhaltsstoffe selektiv dadurch abzutrennen, daß sie in Gasform übergeführt werden und dann direkt oder indirekt - in letzterem Fall mit optischen Analysengeräten, wie beispielsweise einem Photometer - analysiert werden. Dies geschieht insbesondere zur Bestimmung von Kohlenstoff, wobei der gesamte organische Kohlenstoff (TOC) von besonderem Interesse ist. Die Bestimmung des TOC geschieht dabei grundsätzlich nach der DIN EN 1484.

Die Aufbereitung des Probeninhaltsstoffs geschieht grundsätzlich in der Weise, daß das TOC - nach vorheriger Entfernung des anorganischen Kohlenstoffs (TIC) - mittels eines Oxidationsmittels, beispielsweise Natriumperoxodisulfat, in Gasform, d.h. in CO₂, umgesetzt wird und aus dem Probenaufnahmebehälter mittels eines inerten Trägergases, beispielsweise durch Wasserdampfdestillation, oder durch überschüssiges Reaktionsgas, in einen Analysenbehälter getrieben wird. Dort wird das CO₂ in einem flüssigen oder in Festform vorliegenden Indikatorreagenz absorbiert. Das Indikatorreagenz erfährt hierdurch eine optische Veränderung, die in einem optischen Analysengerät, beispielsweise einem Photometer, analysiert werden kann.

Um diese Analytik einfach und schnell vor Ort durch wenig vorgebildetes Personal und mit preiswerten Mitteln durchführen zu können, sind Testkits entwickelt worden, wie sie beispielsweise in der EP 0 663 239 B1 beschrieben sind. Dieser Testkit hat zwei als Glasküvetten ausgebildete Behälter, nämlich einen Probeaufnahmebehälter und einen Analysenbehälter, die jeweils obenseitig Behälteröffnungen aufweisen, die mit aufschraubbaren Verschlußkappen verschließbar sind. Zu dem Testkit gehört auch ein Adapter, über den die Behälteröffnungen nach Abnahme der Verschlüsse gasdicht miteinander verbindbar sind. Der Adapter ist mit einer semipermeablen Membran versehen, die für Gase und hier insbesondere den zu analysierenden Inhaltsstoff und das Trägergas durchlässig ist. Hierzu kann sie beispielsweise aus hydrophobem Material bestehen. Der Analysenbehälter kann das Indikatorreagenz in vorkonfektionierter und lagerfähiger Form enthalten. Ebenso kann auch der Probeaufnahmebehälter schon vorkonfektioniert mit einem Aufschlußreagenz versehen sein, der die Überführung des zu analysierenden Inhaltsstoffes in die Gasform bewirkt.

Bei dem bekannten Testkit erfolgt die Absorption des aus der Probe ausgetriebenen Inhaltsstoffes innerhalb eines geschlossenen Systems, bestehend aus den beiden Glaskuvetten und den die Behälteröffnungen gasdicht verbindenden Adapter. Auf diese Weise wird jede Verfälschung durch Lufteintritt von außen vermieden, was wegen des CO₂-Gehalts in der Luft insbesondere bei der TOC-Bestimmung zu unrichtigen Ergebnissen führen würde. Von Nachteil ist jedoch, daß sich in dem Analysenbehälter ein Gegendruck aufbaut, der dem Gasaustausch vom Probeaufnahmebehälter in den Analysenbehälter entgegenwirkt. und auch die Intensität des Farbumschlages an dem Indikatorreagenz behindert.

In der US-5,979,219 ist eine Meßeinrichtung zur Messung von flüchtigen Bestandteilen in einer wässrigen Lösung offenbart. Diese Meßeinrichtung ist dazu bestimmt, in der industriellen Prozeßtechnik zur kontinuierlichen Messung ohne die Verwendung eines Trägergases eingesetzt zu werden. Die Meßeinrichtung weist eine Meßkammer auf, deren Eintritt mit einer gaspermeablen Membran verschlossen ist, über die das zu messende Gas von der Flüssigkeit getrennt wird. In der Meßkammer befindet sich ein Gassensor auf Halbleiterbasis, dessen Widerstand sich in Abhängigkeit der Konzentration des zu messenden Gases verändert. Um eine bessere Meßgenauigkeit, eine höhere Durchflußrate sowie einen problemfreien Betrieb innerhalb eines industriellen Prozesses mit kontinuierlicher Messung zu erhalten, ist die Meßkammer mit einem Auslaß versehen, der ständig Verbindung zur Außenatmosphäre hat und der so bemessen ist, daß der Volumenstrom des durch die Membran in die Meßkammer gehenden Gases größer ist als des die Meßkammer verlassenden Gasstroms. Hierdurch soll eine Aufkonzentrierung des Gases in der Meßkammer innerhalb eines kontinuierlichen Prozesses bewirkt werden.

Der Erfindung liegt die Aufgabe zugrunde, ein vor allem für die Anwendung bei Testkits geeignetes Verfahren so auszugestalten, daß es schneller und quantitativer arbeitet und zu einer intensiveren optischen Änderung des Indikatorreagenzes führt. Eine weitere Aufgabe besteht darin, einen für die Durchführung dieses Verfahrens geeigneten Testkit bereitzustellen.

Die erste Aufgabe wird erfindungsgemäß dadurch gelöst, daß der Analysenbehälter selektiv mit der Außenatmosphäre derart in Verbindung gesetzt wird, daß ein Druckausgleich stattfindet. Grundgedanke der Erfindung ist es also, das Verfahren nicht mehr - wie bekannt - in einem geschlossenen System durchzuführen, sondern den Analysenbehälter während der Austreibung des Inhaltsstoffes zur Atmosphäre hin selektiv zu öffnen und damit einen teilweisen oder vollständigen Druckausgleich mit der Außenatmosphäre herzustellen. Dies beschleunigt den Übertritt des gasförmigen Inhaltsstoffes und des Trägergases in den Analysenbehälter und führt auch zu einer besseren Absorption des Inhaltsstoffes an dem Indikatorreagenz. Außerdem ist hierdurch gesichert, daß der Inhaltsstoff vollständig in den Analysenbehälter gelangt. Letztendlich bildet die Verbindung zur Außenatmosphäre auch eine Art Sicherheitsventil, das ein Platzen der Behälter in Folge von Überdruck vermeidet.

In Ausbildung der Erfindung ist vorgesehen, daß die Verbindung zur Außenatmosphäre erst nach Anbringung des Adapters am Analysenbehälter hergestellt wird. Dies kann beispielsweise durch Entfernung einer die Verbindung sperrenden Abdeckung geschehen.

Für die Herstellung des Druckausgleichs kann der Analysenbehälter beispielsweise mit einem Belüftungsröhrchen durchstochen werden.

Nach der Erfindung ist ferner vorgesehen, daß der Probeaufnahmebehälter nach Einfüllen der Probe derart erhitzt wird, daß ein nicht zu analysierender Inhaltsstoff ausgetrieben wird, beispielsweise anorganischer Kohlenstoff. Um dies zu unterstützen, sollte der Probeaufnahmebehälter vor dem Erhitzen mit einem Austreibreagenz versehen werden, der das Austreiben fördert. Die Erhitzung des Probeaufnahmebehälters kann in an sich bekannten Heizblöcken geschehen.

Der zweite Teil der Aufgabe, der sich auf den Testkit selbst bezieht, wird erfindungsgemäß dadurch gelöst, daß der Analysenbehälter eine Druckentlastungseinrichtung aufweist, über die der Analaysenbehälter selektiv mit der Außenatmosphäre derart in Verbindung bringbar ist, daß ein Druckausgleich stattfindet. Dabei sollte die Druckentlastungseinrichtung vorzugsweise am der Behälteröffnung gegenüberliegenden Ende des Analysenbehälters angeordnet sein. Die Druckentlastungseinrichtung ist zweckmäßigerweise ausschließlich für Gase durchlässig, vor allem wenn als Indikatorreagenz eine Flüssigkeit zur Anwendung kommt. Überschüssiges Trägergas entweicht durch die Druckentlastungseinrichtung und verhindert hierdurch das Eintreten von Luft in den Analysenbehälter.

Die Druckentlastungseinrichtung kann auf vielfältige Weise ausgebildet sein. Besonders einfach ist sie, wenn sie als mit einer semipermeablen Abdeckung geschlossene Behälterdurchbrechung ausgebildet ist, wobei die Abdeckung vorzugsweise aus einem hydrophoben Material besteht. Zweckmäßigerweise ist die Abdeckung als Membran aus beispielsweise PTFE, PVDF oder FEP ausgebildet.

Die Druckentlastungseinrichtung kann alternativ dazu als mit einer durchstechbaren Abdeckung geschlossene Behälterdurchbrechung ausgebildet sein, beispielsweise in Form einer Membran aus Gummi, insbesondere aus Butylkautschuk mit ein- oder beidseitiger Kaschierung mittels PTFE oder FEP. Zum Durchstechen sollte ein zur Druckentlastungseinrichtung gehörendes Belüftungsröhrchen verwendet werden, dessen Innendurchmesser sehr eng bemessen ist. Da die Membran selbstschließend ist, verhindert sie nach dem Herausziehen des Belüftungsröhrchens ein Ausfließen des Indikatorreagenzes.

In weiterer Ausgestaltung der Erfindung ist vorgesehen, daß die Abdeckung der Behälterdurchbrechung außenseitig mit einem abnehmbaren oder abziehbaren Schutzelement versehen ist, beispielsweise in Form einer aufgeklebten Schutzfolie. Dies vermeidet einen Gasaustausch während der Lagerung und des Transports des Testkits.

Was den Adapter selbst angeht, ist in an sich bekannter Weise vorgesehen, daß er mit einer nur für Gase durchlässigen Trennmembran versehen ist, damit ein Flüssigkeitsaustausch zwischen den beiden Behältern vermieden wird. Hierzu kann die Trennmembran beispielsweise aus einem hydrophoben Material bestehen.

In der Zeichnung ist die Erfindung anhand eines Ausführungsbeispiels für den Testkit und durch Darstellung des Verfahrensablaufes näher veranschaulicht. Es zeigen:
- Figur 1: einen Probeaufnahmebehälter, eingestellt in einen Thermoblock;
- Figur 2: den Probeaufnahmebehälter bei dem Einfüllen eines Aufschlußreagenzes;
- Figur 3: einen Analysenbehälter mit aufgeschraubtem Adapter, zusätzlich mit einer vergrößerten Schnittdarstellung;
- Figur 4: den Analysenbehälter gemäß Figur 3 in um 180° verdrehter Stellung zusätzlich mit zwei Versionen von Druckentlastungseinrichtungen in vergrößerter Schnittdarstellung;
- Figur 5: die Kombination von Probeaufnahmebehälter und Analysenbehälter, verbunden durch den Adapter;
- Figur 6: die Kombination gemäß Figur 5, eingestellt in den Thermoblock mit vergrößerter Schnittdarstellung des Adapters;
- Figur 7: die Ansicht der Kombination aus Figur 5 zusammen mit deren Darstellung in um 180° verdrehter Stellung und
- Figur 8: die Kombination aus Figur 7, eingestellt in ein Photometer.

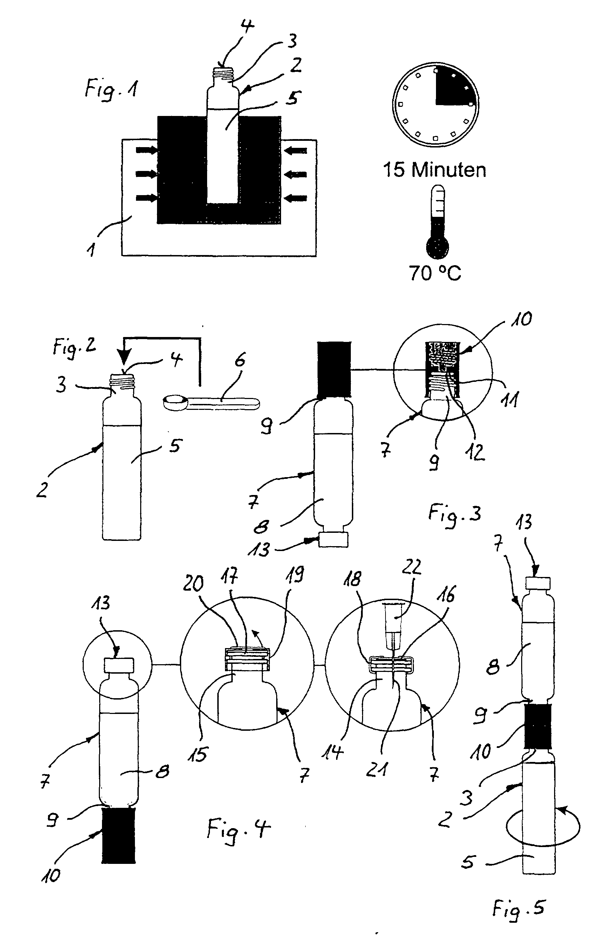

In Figur 1 ist schematisch ein Thermoblock 1 üblicher Bauart im Vertikalschnitt dargestellt. In den Thermoblock 1 ist ein als Rundküvette aus Glas ausgebildeter Probeaufnahmebehälter 2 eingesetzt. Der Probeaufnahmebehälter 2 hat obenseitig einen mit einem Außengewinde versehenen Behälterstutzen 3, der eine Behälteröffnung 4 umgibt.

In dem Probeaufnahmebehälter 2 ist eine Wasserprobe 5 eingefüllt, die mit einer Säuremischung, nämlich Natriumhydrogensulfat, versetzt ist. Die Säuremischung war zuvor in dem Probeaufnahmebehälter 2 vorkonfektioniert enthalten. In dem Thermoblock 1 wird durch Erhitzen auf 70°C über 15 min - symbolisiert durch die rechtseitige Graphik - anorganischer Kohlenstoff ausgetrieben.

Nach Entfernung des anorganischen Kohlenstoffes wird der Probeaufnahmebehälter 2 aus dem Thermoblock 1 herausgenommen. Wie in Figur 2 zu sehen ist, wird dann über die Behälteröffnung 4 mit einem Meßlöffel 6 eine Aufschlußreagenz in Form eines Oxidationsmittels, hier Peroxodisulfat, eingegeben.

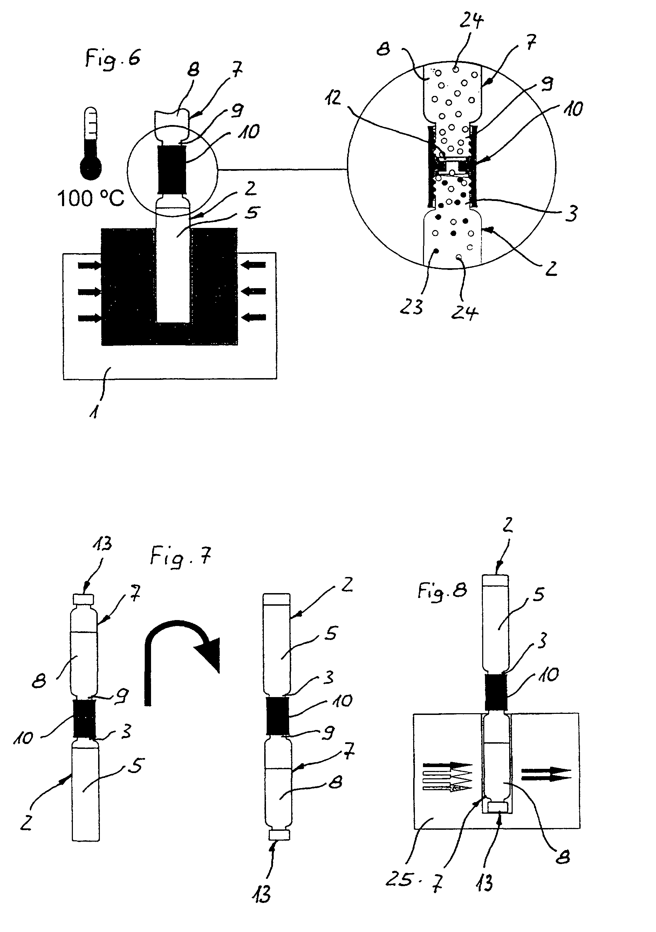

Figur 3 zeigt einen Analysenbehälter 7, der ebenfalls als Rundküvette aus Glas ausgebildet ist und in dem ein flüssiges Indikatorreagenz 8 eingefüllt ist. Der Analysenbehälter 7 hat obenseitig einen Behälterstutzen 9, der auch hier eine Behälteröffnung umschließt. Auf den Behälterstutzen 9 ist ein Adapter 10 aus Kunststoff aufgeschraubt. Dessen Aufbau ergibt sich aus der vergrößerten Vertikalschnittdarstellung im rechten Teil der Figur. Er hat die Form einer Hülse, die mittig einen innenseitig vorspringenden Ringsteg 11 aufweist, wobei oberhalb und unterhalb des Ringsteges 11 Innengewinde eingeformt sind. Über das untenseitige Innengewinde ist der Adapter 10 auf den Behälterstutzen 9 aufgeschraubt. Zwischen Stirnseite des Behälterstutzens 9 und Unterseite des Ringstegs 10 ist eine hydrophobe Trennmembran 12 eingeklemmt, die für Gase durchlässig ist, nicht jedoch für Flüssigkeiten und damit auch nicht für das Indikatorreagenz 8.

An der Unterseite des Analysenbehälters 7 befindet sich eine Druckentlastungseinrichtung 13. Zwei mögliche Ausführungsformen dieser Druckentlastungseinrichtung 13 sind in Figur 4 dargestellt. Dort ist der Analysenbehälter 7 um 180° gedreht gezeigt, so daß sich der Adapter 10 unten und die Druckentlastungseinrichtung 13 oben befinden.

Die beiden Ausführungsformen der Druckentlastungseinrichtung 13 sind rechtsseitig durch Kreise eingerahmt und vergrößert dargestellt. Beide Druckentlastungseinrichtungen 13 sind an einem Entlastungsstutzen 14, 15 angebracht, der Teil des Analysenbehälters 7 ist. Die Entlastungsstutzen 14, 15 sind obenseitig durch Membranen 16, 17 abgedeckt, die durch Klammerringe 18, 19 fest und damit gasdicht auf die Stirnseiten der Entlastungsstutzen 14, 15 aufgedrückt und so fixiert werden. Die Membranen 16, 17 sind flüssigkeitsundurchlässig, so daß das Indikatorreagenz 8 nicht auslaufen kann, selbst wenn sich der Analysenbehälter nicht in der in Figur 3 gezeigten Stellung befindet.

Bei dem linksseitigen Ausführungsbeispiel ist die Membran 17 semipermeabel. Sie ist für das Trägergas, das in dem Probeaufnahmebehälter 2 nach Eingabe des Aufschlußreagenzes (Figur 2) entsteht, durchlässig. Die von dem Klammerring 19 freigelassene Öffnung ist außenseitig mit einer Klebefolie 20 abgedeckt, die die Membran 17 in der Zeit vor der Benutzung des Analysenbehälters 7 schützt und eine gasdichte Barriere bildet. Vor Gebrauch des Analysenbehälters 7 wird die Klebefolie 20 von Hand abgezogen, was durch den eingezeichneten Pfeil symbolisiert ist. Die Porenweite der Membran 17 liegt im Bereich von unter 50 µm, vorzugsweise bei 20 µm.

In dem rechtsseitigen Ausführungsbeispiel besteht die Membran 16 aus Gummi, ist also flüssigkeits- und gasdicht. Zum Zweck der Verbindung zur Außenatmosphäre wird in die Membran 16 ein nadelförmiges Belüftungsröhrchen 21 eingestochen, dessen Innendurchmesser 0,45 mm beträgt. Das Belüftungsröhrchen 21 steckt mit seinem außenseitigen Ende in einer Handhabungshülse 22.

Der Analysenbehälter 7 kann schon in dem Testkit in der in Figur 3 gezeigten Form vorkonfektioniert, also mit dem Indikatorreagenz 8 und dem Adapter 10 versehen sein. Es besteht jedoch auch die Möglichkeit, den Analysenbehälter 7 erst dann mit dem Indikatorreagenz 8 zu befüllen und den Adapter 10 aufzuschrauben, wenn eine Analyse durchgeführt wird, und zwar zweckmäßigerweise parallel zu der Eingabe des Aufschlußreagenzes in den Probeaufnahmebehälter 2.

Der Probeaufnahmebehälter 2 sollte unmittelbar nach Eingabe des Aufschlußreagenz mit dem Adapter 10 verschraubt werden, wie dies in Figur 5 dargestellt ist. Der Analysenbehälter 7 hat dabei die in Figur 4 dargestellte Stellung mit untenseitigem Adapter 10, und der Probeaufnahmebehälter 2 wird dann von unten in den Adapter 10 eingeschraubt. Der Adapter 10 verbindet die Behälteröffnungen 4 von Probeaufnahmebehälter 2 und Analysenbehälter 7 abdichtend. Die Öffnung der Druckentlastungseinrichtung 13 im oben beschriebenen Sinn kann vor oder auch unmittelbar nach dem Anschrauben des Probeaufnahmebehälters 2 geschehen.

Anschließend wird die aus Probeaufnahmebehälter 2, Analysenbehälter 7 und Adapter 10 bestehende Einheit wieder in den Thermoblock 1 eingestellt, wobei jedoch nur der Probeaufnahmebehälter 2 in den Thermoblock 1 hineinragt. Dies ist in Figur 6 dargestellt. In dem Thermoblock 1 wird die mit dem Aufschlußreagenz versehene Wasserprobe 5 auf 100°C erhitzt. Das Aufschlußreagenz und die Wärme bewirken zweierlei. Zum einen wird der noch vorhandene organische Kohlenstoff vergast und aus der Wasserprobe 5 ausgetrieben. Zum anderen entsteht ein Trägergas 24 aus Sauerstoff und Wasserdampf, das als Träger für das CO₂ 23 dient und dieses aufgrund des entstehenden Überdrucks nach oben in Richtung auf den Analysenbehälter 7 mitnimmt. CO₂ 23 und Trägergas 24 durchströmen die Trennmembran 12 und gelangen in das Indikatorreagenz 8. Dort wird das CO₂ 23 absorbiert und führt zu einer optischen Veränderung, beispielsweise einem Farbumschlag des Indikatorreagenzes 8.

Das Trägergas 24 durchperlt das Indikatorreagenz 8, wobei es durchmischt wird, und kann anschließend über die Druckentlastungseinrichtung 13 in die Außenatmosphäre entweichen. Hierdurch wird ein Druckaufbau in dem Analysenbehälter 7 vermieden. Dies beschleunigt nicht nur den Gastransport in den Analysenbehälter 7, sondern sichert auch den vollständigen Übergang des CO₂ 23 in das Indikatorreagenz 8. Das intensive Durchperlen des Trägergases 24 erhöht zudem die Kontaktfläche und hat hierdurch einen intensiveren Farbumschlag zur Folge. Letztendlich wird durch die Druckentlastungseinrichtung 13 auch ein Platzen des Probeaufnahmebehälters 2 oder des.Analysenbehälters 7 vermieden.

Nach Abschluß des Austreibens des CO₂ 23 wird die Einheit aus Probeaufnahmebehälter 2 und Analysenbehälter 7 aus dem Thermoblock 1 herausgenommen und - wie Figur 7 zeigt - um 180° gedreht, so daß sich der Probeaufnahmebehälter 2 wieder oben und der Analysebehälter 7 erneut unten befinden. Bei dem in Figur 4 rechtsseitigen Ausführungsbeispiel muß zuvor das Belüftungsröhrchen 21 entfernt werden, wobei sich die Membran 16 selbstätig flüssigkeitsdicht schließt. In dieser Stellung wird die Einheit in ein Photometer 25 eingestellt, wobei jedoch nur der Analysenbehälter 7 in das Photometer 25 hineinragt. Dort wird der Analysenbehälter 7 und das Indikatorreagenz 8 - symbolisiert durch die Pfeile - mit Licht eines bestimmten Wellenlängenbereichs durchstrahlt und die durch das Indikatorreagenz 8 erzeugte Extinktion als Maß für die Menge des durch das Indikatorreagenz absorbierten Inhaltsstoffes, hier CO₂, erfaßt.

## Patentansprüche

1. Verfahren für die Analyse eines gasförmigen oder in Gasform überführbaren Inhaltsstoffes (23) einer Probe (5), bei dem die Probe (5) über eine Behälteröffnung (4) in einen Probeaufnahmebehälter (2) eingefüllt und ein zuvor mit einem Indikatorreagenz (8) versehener Analysenbehälter (7) mit seiner Behälteröffnung über einen Adapter (10) mit der Behälteröffnung (4) des Probeaufnahmebehälters (2) verbunden wird, wonach der Inhaltsstoff (23) aus dem Probeaufnahmebehälter (2) in den Analysenbehälter (7) getrieben wird, **dadurch gekennzeichnet, daß** der Analysenbehälter (7) selektiv mit der Außenatmosphäre derart in Verbindung gesetzt wird, daß ein Druckausgleich stattfindet.

2. Verfahren nach Anspruch 1, **dadurch gekennzeichnet, daß** die Verbindung zur Außenatmosphäre erst nach Anbringen des Adapters (10) am Analysenbehälter hergestellt wird.

3. Verfahren nach Anspruch 1 oder 2, **dadurch gekennzeichnet, daß** der Analysenbehälter (7) für den Druckausgleich mit einem Belüftungsröhrchen (21) durchstochen wird.

4. Verfahren nach einem der Ansprüche 1 bis 3, **dadurch gekennzeichnet, daß** der Probeaufnahmebehälter (2) nach Einfüllen der Probe (5) derart erhitzt wird, daß ein nicht zu analysierender Inhaltsstoff ausgetrieben wird.

5. Verfahren nach Anspruch 4, **dadurch gekennzeichnet, daß** der Probeaufnahmebehälter (2) vor dem Erhitzen mit einem Austreibreagenz versehen wird, der das Austreiben des nicht zu analysierenden Inhaltsstoffes fördert.

6. Testkit für die Analyse eines gasförmigen oder in Gasform überführbaren Inhaltsstoffes (23) einer Probe (5) mit einem Probeaufnahmebehälter (2) für die Aufnahme der Probe (5) über eine Behälteröffnung (4) und mit einem Analysenbehälter (7) für die Aufnahme des zu analysierenden Inhaltsstoffes (23) über eine Behälteröffnung, wobei der Analysenbehälter (7) ein Indikatorreagenz (8) enthält oder mit einem Indikatorreagenz (8) versehbar und als Meßvorlage in einem optischen Meßgerät (25) verwendbar ist, sowie mit einem Adapter (10), über den die Behälteröffnungen (4) miteinander verbindbar sind, **dadurch gekennzeichnet, daß** der Analysenbehälter (7) eine Druckentlastungseinrichtung (13) aufweist, über die der Analysenbehälter (7) selektiv mit der Außenatmosphäre derart in Verbindung bringbar ist, daß ein Druckausgleich stattfindet.

7. Testkit nach Anspruch 6, **dadurch gekennzeichnet, daß** die Druckentlastungseinrichtung (13) an dem der Behälteröffnung (4) gegenüberliegenden Ende des Analysenbehälters (7) angeordnet ist.

8. Testkit nach Anspruch 7, **dadurch gekennzeichnet, daß** die Druckentlastungseinrichtung (13) ausschließlich für Gase durchlässig ist.

9. Testkit nach einem der Ansprüche 6 bis 8, **dadurch gekennzeichnet, daß** die Druckentlastungseinrichtung (13) als mit einer semipermeablen Abdeckung (17) geschlossenen Behälterdurchbrechung (15) ausgebildet ist.

10. Testkit nach Anspruch 9, **dadurch gekennzeichnet, daß** die Abdeckung (17) aus hydrophobem Material besteht.

11. Testkit nach Anspruch 9 oder 10, **dadurch gekennzeichnet, daß** die Abdeckung (17) als Membran aus z.B. PTFE, PVDF oder FEP ausgebildet ist.

12. Testkit nach einem der Ansprüche 6 bis 8, **dadurch gekennzeichnet, daß** die Druckentlastungseinrichtung (13) als mit einer durchstechbaren Abdeckung (16) geschlossene Behälterdurchbrechung (14) ausgebildet ist.

13. Testkit nach Anspruch 12, **dadurch gekennzeichnet, daß** die Abdeckung als Membran (16) ausgebildet ist.

14. Testkit nach Anspruch 13, **dadurch gekennzeichnet, daß** die Membran aus Gummi, insbesondere aus Butylkautschuk, vorzugsweise mit PTFE, oder FEP beschichtet, besteht.

15. Testkit nach einem der Ansprüche 12 bis 14, **dadurch gekennzeichnet, daß** zur Druckentlastungseinrichtung (13) ein Belüftungsröhrchen (21) gehört, das durch die Abdeckung (16) stechbar ist.

16. Testkit nach einem der Ansprüche 9 bis 15, **dadurch gekennzeichnet, daß** die Abdeckung (17, 18) außenseitig mit einem abnehmbaren oder abziehbaren Schutzelement (20) versehen ist.

17. Testkit nach Anspruch 16, **dadurch gekennzeichnet, daß** das Abdeckelement als aufgeklebte Schutzfolie (20). ausgebildet ist.

18. Testkit nach einem der Ansprüche 6 bis 17, **dadurch gekennzeichnet, daß** die Adapter (10) mit einer für Gase durchlässigen Trennmembran (12) versehen ist.

19. Testkit nach Anspruch 18, **dadurch gekennzeichnet, daß** die Trennmembran (12) aus einem hydrophoben Material besteht.

## Claims

1. Method for the analysis of a gaseous component or of a component that can be transformed into gaseous form (23) of a sample (5) which provides that the sample (5) is filled into a sample receptacle (2) through a receptacle opening (4), and an analysis receptacle (7), which was previously supplied with a tracer reagent, is then by way of its receptacle opening (4) connected with the receptacle opening (4) of sample receptacle (2) via an adapter (10), after which step the component (23) is expelled from the sample receptacle (2) and into the analysis receptacle (7) **characterized in that** the analysis receptacle (7) is selectively connected with the outside atmosphere in such a manner that a pressure compensation takes place.

2. Method as claimed in claim 1 **characterized in that** the connection with the outside atmosphere is only established after the adapter (10) has been attached to the analysis receptacle (7).

3. Method as claimed in claim 1 or in claim 2 **characterized in that** for the pressure compensation the analysis receptacle (7) is perforated with a ventilation tube.

4. Method as claimed in one of the claims 1 to 3 **characterized in that** the sample receptacle (2) is heated after the sample (5) has been filled in such a way that a component that is not to be analyzed is expelled.

5. Method as claimed in claim 4 **characterized in that** before being heated the sample receptacle (2) is supplied with an expulsion reagent which supports the expulsion of the component that is not to be analyzed.

6. Test kit for the analysis of a gaseous component or of a component that can be transformed into gaseous form (23) of a sample (5) comprising a sample receptacle (2) for receiving the sample (5) through a receptacle opening (4) and an analysis receptacle (7) for receiving the component that is to be analyzed (23) through a receptacle opening (4), wherein the analysis receptacle (7) contains a tracer reagent (8) or can be supplied with a tracer reagent (8) and can be used as a measuring receiving flask in an optical measuring instrument (25), and an adapter (10) which is used to connect the receptacle openings (4) with each other, **characterized in that** the analysis receptacle (7) is equipped with a pressure relief device (13), whereby the analysis receptacle (7) can be selectively connected with the outside atmosphere in such a manner that a pressure compensation takes place.

7. Test kit as claimed in claim 6 **characterized in that** the pressure relief device (13) is arranged on the end of the analysis receptacle (7) that is opposite to the receptacle opening (4).

8. Test kit as claimed in claim 7 **characterized in that** the pressure relief device (13) is only permeable for gases.

9. Test kit as claimed in one of the claims 6 to 8 **characterized in that** the pressure relief device (13) is realized as a receptacle opening (15) that is closed off with a semi-permeable covering (17).

10. Test kit as claimed in claim 9 **characterized in that** the covering (17) consists of a hydrophobic material.

11. Test kit as claimed in claim 9 or claim 10 **characterized in that** the covering (17) is realized as a membrane consisting of e.g. PTFE, PVDF or FEP.

12. Test kit as claimed in one of the claims 6 to 8 **characterized in that** the pressure relief device (13) is realized as a receptacle opening (14) that is closed off with a covering (16) that can be perforated.

13. Test kit as claimed in claim 12 **characterized in that** the covering is realized as a membrane (16).

14. Test kit as claimed in claim 13 **characterized in that** the membrane consists of rubber, in particular of isobutylene-isoprene copolymer, preferably coated with PTFE or FEP.

15. Test kit as claimed in one of the claims 12 to 14 **characterized in that** a ventilation tube (21) that can be pierced through the covering (16) forms part of the pressure relief device (13).

16. Test kit as claimed in one of the claims 9 to 15 **characterized in that** on its outer side the covering (17, 18) is equipped with a removable or pull-off protective element (20).

17. Test kit as claimed in claim 16 **characterized in that** the covering element is realized as a paste-on protective foil (20).

18. Test kit as claimed in one of the claims 6 to 17 **characterized in that** the adapter (10) is equipped with a separating membrane (12) that is permeable for gases.

19. Test kit as claimed in claim 18 **characterized in that** the separating membrane (12) consists of a hydrophobic material.

## Revendications

1. Procédé d'analyse d'un contenu (23) gazeux ou transformable en une forme gazeuse d'un échantillon (5), dans laquelle l'échantillon (5) est versé par une ouverture de récipient (4) dans un récipient récepteur d'échantillons (2) et dans laquelle un récipient d'analyse (7), équipé au préalable d'un réactif indicateur (8), est relié par son ouverture de récipient, via un adaptateur (10), à une ouverture de récipient (4) du récipient récepteur d'échantillons (2), suite à quoi le contenu (23) est entraîné du récipient récepteur d'échantillons (2) dans le récipient d'analyse (7), **caractérisée en ce que** le récipient d'analyse (7) est relié de façon sélective à l'atmosphère externe de façon à ce qu'il se produise une compensation de pression.

2. Procédé selon la revendication 1, **caractérisée en ce que** la liaison à l'atmosphère externe est réalisée uniquement après application de l'adaptateur (10) sur le récipient d'analyse.

3. Procédé selon la revendication 1 ou 2, **caractérisé en ce que** le récipient d'analyse (7) est traversé, pour la compensation de pression, par une tubulure d'aération (21).

4. Procédé selon l'une des revendications 1 à 3, **caractérisé en ce que** le récipient récepteur d'échantillons (2) est chauffé, après introduction de l'échantillon (5), de façon telle qu'un contenu qui n'est pas à analyser est expulsé.

5. Procédé selon la revendication 4, **caractérisé en ce que** le récipient récepteur d'échantillons (2), avant le chauffage, est pourvu d'un réactif d'expulsion qui favorise l'expulsion du contenu qui n'est pas à analyser.

6. Trousse d'essai pour l'analyse d'un contenu (23) gazeux ou transformable en une forme gazeuse d'un échantillon (5), comportant un récipient récepteur d'échantillons (2) destiné à recevoir l'échantillon (5) par une ouverture de récipient (4), et un récipient d'analyse (7) destiné à recevoir le contenu à analyser (23) par une ouverture de récipient, dans laquelle le récipient d'analyse (7) contient un réactif indicateur (8) ou peut être pourvu d'un réactif indicateur (8) et peut être utilisé comme support de mesure dans un appareil de mesure optique (25), ainsi qu'un adaptateur (10) par lequel les ouvertures de récipient (4) peuvent être reliées entre elles, **caractérisée en ce que** le récipient d'analyse (7) présente un dispositif de décharge de la pression (13) par lequel le récipient d'analyse (7) peut être amené sélectivement en liaison avec l'atmosphère externe de façon à ce qu'il se forme une compensation de pression.

7. Trousse d'essai selon la revendication 6, **caractérisée en ce que** le dispositif de décharge de la pression (13) est disposé au niveau de l'extrémité du récipient d'analyse (7) opposée à l'ouverture de récipient (4).

8. Trousse d'essai selon la revendication 7, **caractérisée en ce que** le dispositif de décharge de la pression (13) est exclusivement perméable aux gaz.

9. Trousse d'essai selon l'une des revendications 6 à 8, **caractérisée en ce que** le dispositif de décharge de la pression (13) est formé comme une découpe de récipient (15) fermée par un couvercle semi-perméable (17).

10. Trousse d'essai selon la revendication 9, **caractérisée en ce que** le couvercle (17) se compose d'un matériau hydrophobe.

11. Trousse d'essai selon la revendication 9 ou 10, **caractérisée en ce que** le couvercle (17) est réalisé sous la forme d'une membrane réalisée, par exemple, en PTFE, en PVDF ou en FEP.

12. Trousse d'essai selon l'une des revendications 6 à 8, **caractérisée en ce que** le dispositif de décharge de la pression (13) est formé comme une découpe de récipient (14) fermée par un couvercle (16) perforable.

13. Trousse d'essai selon la revendication 12, **caractérisée en ce que** le couvercle est réalisé sous la forme d'une membrane (16).

14. Trousse d'essai selon la revendication 13, **caractérisée en ce que** la membrane est réalisée en caoutchouc, en particulier en caoutchouc butylé, de préférence revêtu de PTFE ou de FEP.

15. Trousse d'essai selon l'une des revendications 12 à 14, **caractérisée en ce qu'**une tubulure d'aération (21), qui peut traverser en le perçant, le couvercle (16) appartient au dispositif de décharge de la pression (13).

16. Trousse d'essai selon l'une des revendications 9 à 15, **caractérisée en ce que** le couvercle (17, 18) est pourvu, sur le côté externe, d'un élément protecteur (20) amovible ou pelable.

17. Trousse d'essai selon la revendication 16, **caractérisé en ce que** l'élément de couvercle est réalisé sous la forme d'une feuille protectrice (20) collée.

18. Trousse d'essai selon l'une des revendications 6 à 17, **caractérisée en ce que** l'adaptateur (10) est équipé d'une membrane de séparation (12) perméable aux gaz.

19. Trousse d'essai selon la revendication 18, **caractérisée en ce que** la membrane de séparation (12) est réalisée en un matériau hydrophobe.
